Europäisches Patentamt

European Patent Office

Office européen des brevets

(19)

(11) Veröffentlichungsnummer: **0 334 278**

**A2**

(12) # EUROPÄISCHE PATENTANMELDUNG

(21) Anmeldenummer: 89105006.4

(22) Anmeldetag: 21.03.89

(51) Int. Cl.4: **C07K 15/04** , //A61K39/02

(30) Priorität: 23.03.88 DE 3809796

(43) Veröffentlichungstag der Anmeldung:
27.09.89 Patentblatt 89/39

(84) Benannte Vertragsstaaten:
AT BE CH DE FR GB IT LI LU NL SE

(71) Anmelder: **Bredt, Wolfgang, Prof. Dr.**
**Sickingenstrasse 70**
**D-7800 Freiburg(DE)**

Anmelder: **Fuchte, Klemens, Dr.**
**Georgstrasse 23**
**D-5100 Aachen Brandt(DE)**

Anmelder: **Jacobs, Enno, Dr.**
**Schlehenrain 10**
**D-7800 Freiburg(DE)**

(72) Erfinder: **Bredt, Wolfgang, Prof. Dr.**
**Sickingenstrasse 70**
**D-7800 Freiburg(DE)**
Erfinder: **Fuchte, Klemens, Dr.**
**Georgstrasse 23**
**D-5100 Aachen Brandt(DE)**
Erfinder: **Jacobs, Enno, Dr.**
**Schlehenrain 10**
**D-7800 Freiburg(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) Verfahren zum Reinigen eines 168 kD Proteins von Mycoplasma pneumoniae.

(57) Die Erfindung bezieht sich auf ein Verfahren zum Reinigen eines 168 kD-Proteins von Mycoplasma pneumoniae.

Es war bisher nicht möglich, im Stand der Technik das 168 kD-Protein von Mycoplasma pneumoniae in einer Reinheit zur Verfügung zu stellen, die für die diagnostische oder therapeutische Verwendung des Proteins ausreichend gewesen wäre.

Es wär daher Aufgabe der Erfindung, ein Verfahren zur Verfügung zu stellen, das das 168 kD-Protein von Mycoplasma pneumoniae in ausreichenden Mengen in nativer Form zur Verfügung stellt.

Diese Aufgabe wird dadurch gelöst, daß ganze Mycoplasma pneumoniae Zellen mit einem zwitterionischen Detergenz, bevorzugt CHAPS, behandelt werden, sodann aus dem erhaltenen Pellet das 168 kD-Protein mit einem nonionischen Detergenz, bevorzugt Octylglucosid, extrahiert und schließlich weitere Verunreinigungen durch Größenausschlußchromatographie entfernt werden.

## Verfahren zum Reinigen eines 168 kD-Proteins von Mycoplasma pneumoniae

Die Erfindung bezieht sich auf ein Verfahren zum Reinigen eines 168 kD Proteins von Mycoplasma pneumoniae.

Mycoplasma pneumoniae verursacht Erkrankungen des oberen und unteren Respirationstraktes. Bis zu 10% aller Pharyngitiden, 10-30% aller Pneumonien in einer Zivilbevölkerung und bis ca. 60% der Pneumonien in geschlossenen Populationen können diesem Erreger zugerechnet werden. Epidemien treten in Intervallen von 4-7 Jahren auf und es sind auch Todesfälle in der Literatur beschrieben. (Rollins S, et al., Arch. Pathol. Lab.Med. 110; S.34-41 (1986)). Darüberhinaus sind zahlreiche postinfektiöse Komplikationen bekannt wie Myokarditis, Endokarditis, Meningitis usw., die einer ätiologischen Abklärung bedürfen. Reinfektionen sind keine Seltenheit. Sie treten hauptsächlich in Erwachsenenpopulationen auf, wobei gerade in dieser Gruppe mit klinischen Komplikationen zu rechnen ist.

Da M.pneumoniae ein Bakterium ist und somit im Gegensatz zu Viren, die überwiegend Ursache für Erkrankungen des oberen und unteren Respirationstraktes sind, gegen Antibiotica empfindlich ist, hat eine schnelle Diagnose erhebliche therapeutische Konsequenzen. Klinisch ist jedoch eine sichere Abgrenzung der M.pneumoniae-Infektion von ähnlichen viralen bzw. bakteriellen Infektionen nicht möglich. Ebenso ist die verfügbare Labordiagnostik unbefriedigend. Der Erregernachweis durch Kultur hat wegen des sehr langsamen Wachstums von 1-2 Wochen auf anspruchsvollen Medien nur bestätigenden Wert. In den meisten Fällen wird derzeit die Diagnose serologisch gestellt. Der gebräuchlichste Test, die Komplementbindungsreaktion (KBR), verwendet einen Glykolipidextrakt aus M.pneumoniae als Antigen. Dieses Glycolipid zeigt jedoch Kreuzreaktionen sowohl mit anderen Bakterien, beispielsweise Streptococcus MG intermedius, als auch mit Pflanzenlipiden oder Bestandteilen humaner Zellen.

Insbesondere bei Erkrankungen, die mit Zellzerfall einhergehen, z.B. Pankreatitis, Meningitis oder Carditis, sind falsch-positive KBR-Reaktionen beschrieben worden. Andere Testverfahren sind entweder zu arbeitsaufwendig, wie z.B. Immunfluoreszenz, ebenso wenig spezifisch wie die KBR (ELISA mit M.pneumoniae-Gesamtextrakt (Mitzutani H, Mizutani H (An.Rev.Respir.Dis. 127; S. 175-179, (1983)) oder nur in Speziallaboratorien verwendbar, beispielsweise als Adhärenzinhibitionstest (Jacobs E, et al., Eur.J.Clin.Microbiol. 4; S. 113-118, (1985)).

M.pneumoniae weist in seiner Spitzenstruktur ein Protein mit dem Molekulargewicht (MW) von 168 kD auf, das für die Adhärenz des Erregers an den Zellen des Wirtsorganismus verantwortlich ist (Feldner J. et al., Nature 298; S. 765-769, (1982)). Antikörper gegen dieses Protein konnten in allen bisher untersuchten Seren M.pneumoniae-infizierter Patienten gefunden werden, während die Immunantwort gegen andere Proteine nicht regelmäßig auftrat (Jacobs E, et al., J.Clin./Microbiol.23; S.517-522, (1986a)). Dieses 168 kD Protein ist somit ein geeignetes Antigen für einen spezifischen serologischen Test.

Dieses 168 kD-Protein wurde bereits mit einer Detergenz-Mischung, bestehend aus Triton, DOC, SDS, EDTA, Tris-Puffer (TDSET) isoliert und durch Immunaffinitätschromatographie (Leith D K, Baseman J B., J Bacteriol 157; S. 678-680, Purification of a Mycoplasma pneumoniae adhesin by monoclonal antibody affinity chromatography, (1984)) gereinigt. Das Eluieren des bei der Immunaffinitätschromatographie an die an die Säule gekoppelten Antikörper gebundenen 168 kD-Proteins erfordert jedoch verhältnismäßig harte Bedingungen zum Dissoziieren des Immunkomplexes, die in einem Denaturieren des Liganden und einer Zerstörung der Säule resultieren können. Ein Verfahren, das das Isolieren großer Mengen denaturierten 168 kD-Proteins erlaubt, wurde bei Jacobs und Clad (Jacobs E, Clad A, Anal.Biochem., 154; S. 583-589, Electroelution of fixed and stained membrane proteins from preparative sodium dodecyl sulfate-polyacrylamide gels into a membrane trap (1986)) beschrieben. In dieser Arbeit wurde ein vollständiges M.pneumoniae-Protein durch SDS-PAGE getrennt und markiert und sodann die Bande, die das 168 kD-Protein enthält, aus dem Gel geschnitten und in einem Biotrap-System elektroeluiert. Zwar kann mit diesem Verfahren das 168 kD-Protein in Mengen isoliert werden, die für die Verwendung als Antigen in einem DOT-ELISA zu verwenden sind (Jacobs et al., Eur. J. Clin. Microbiol. 5; S. 435-440, 168-kilodalton protein of Mycoplasma pneumoniae used as antigen in a dot enzyme-linked immunosorbent assay (1986)), das Isolieren des Proteins ist jedoch sehr zeitaufwendig und das mit diesem Verfahren erhaltene Protein weist nicht den nativen Zustand auf.

Es war daher Aufgabe der Erfindung, ein Verfahren zur Verfügung zu stellen, das das 168 kD-Protein von Mycoplasma pneumoniae in ausreichenden Mengen in nativer Form zur Verfügung stellt.

Diese Aufgabe wird, ausgehend von einem Verfahren zum Reinigen eines 168 kD-Proteins von Mycoplasma pneumoniae dadurch gelöst, daß ganze M.pneumoniae-Zellen mit einem zwitterionischen Detergenz, bevorzugt (3-Cholamidopropyl) dimethylammonio-3-Propansulfonat (CHAPS) behandelt werden, sodann aus dem erhaltenen Pellet das 168 kD-Protein mit einem nonionischen Detergenz, bevorzugt Octyl-ß-D-glucopyranosid (Octylglucosid) extrahiert und schließlich weitere Verunreinigungen durch Größenausschlußchromatographie entfernt werden.

Wie bereits erwähnt, verwendet Leith (1984, a.a.O.) für das Lösen den Membranproteine von M.pneumoniae eine Mischung verschiedener Detergenzien, enthaltend Triton X-100, SDS und Deoxycholat (TDSET). Diese Mischung löst jedoch nicht nur das 168 kD-Protein aus der Membran von M.pneumoniae, sondern auch kontaminierende hochmolekulare Proteine, die durch Gelfiltrierung nicht voneinander getrennt werden können. Mit dem erfindungsgemäßen Verfahren war es nun möglich, das 168 kD-Protein durch fraktioniertes Solubilisieren bis zur 20fachen Menge anzureichern. Die verbleibenden Unreinheiten hatten signifikant geringeres Molekulargewicht und konnten durch einen einfachen Gelfiltrationsschritt entfernt werden. Es ist bei Anwendung des erfindungsgemäßen Verfahrens somit nicht nötig, das 168 kD-Protein in denanturierenden Bedingungen auszusetzen, so daß das Protein in überraschend großer Menge in seiner nativen Form zur Verfügung gestellt werden kann.

Die milden Lösungsbedingungen sind auf den Charakter der verwendeten Detergenzien zurückzuführen. Octylglucosid ist ein nichtionisches Detergenz und CHAPS ist ein zwitterionisches Detergenz. Beide Detergenzien haben eine definierte Struktur und einen HLB von etwa 15. Die durch die Verwendung der genannten Detergenzien erhaltenen Ergebnisse wurden verglichen mit Ergebnissen, die nach dem Isolieren aller Zellproteine durch SDS-Puffer oder TDSET erhalten wurden.

Für den Vergleich wurden Zellen mit dem Lösungspuffer, enthaltend entweder CHAPS oder Octylglucosid bzw. mit dem Detergenziengemisch TDSET behandelt. Die erhaltenen Suspensionen wurden zentrifugiert und der erhaltene Überstand in SDS-PAGE analysiert. Die Ergebnisse zeigten, daß TDSET nicht nur für Moleküle geringeren Molekulargewichts gute Löslichkeit aufweist, sondern ebenfalls für das 168 kD-Protein sowie ein weiteres prominentes Protein der Zellmembran mit einem Molekulargewicht von 145 kD. Wie erwartet, konnte durch die Behandlung mit TDSET das nur geringfügig kleinere 145kD-Protein durch anschließende Größenausschluß-Chromatographie von dem 168 kD-Protein nicht getrennt werden.

Octylglucosid zeigte eine gute Löslichkeit für das 168 kD-Protein und eine geringere Löslichkeit für das 145 kD-Protein. Diese Eigenschaft trifft für CHAPS nicht zu, das offensichtlich ein sehr effizientes Detergenz für die Löslichkeit des 145 kD-Proteins, für die Löslichkeit des 168 kD-Proteins jedoch weniger wirksam ist. Die Kombination beider Detergenzien zeigte ein überraschend gutes Ergebnis.

In einem ersten Verfahrensschritt werden die vollständigen Zellen von M.pneumoniae mit CHAPS vorbehandelt, wodurch die Hauptmenge der Proteine, einschließlich des 145 kD-Proteins und eine geringe Menge des 168 kD-Proteins gelöst werden. Nach dem Zentrifugieren des erhaltenen Homogenats werden Aliquots des Pellets mit verschiedenen Konzentrationen von Octylglucosid solubilisiert.

Für den ersten Behandlungsschritt der ganzen Zellen von M.pneumoniae wird bevorzugt ein Puffergemisch (Puffer I) verwendet, in dem das zwitterionische Detergenz CHAPS in ein Konzentration von etwa 0,1-10 % (w/v), bevorzugt 1% (w/v) enthalten ist.

Die Behandlung des nach dem ersten Lösungsschritt erhaltenen Pellets mit dem nonionischen Detergenz Octylglucosid erfolgt ebenfalls bevorzugt mit einem Puffergemisch (Puffer II), in dem Octylglucosid in einer Konzentration von etwa 0,1-10% (w/v), bevorzugt 1-2% (w/v) enthalten ist.

Die erfindungsgemäße, fraktionierte Solubilisierung des 168 kD-Proteins resultiert in einer 20fachen Anreicherung des gewünschten Proteins, verglichen mit der Menge, wie sie natürlicherweise in M.pneumoniae vorkommt, die bei etwa 1,5% des gesamten Zellproteins liegt. Der weitere enorme Vorteil des beschriebenen, erfindungsgemäßen Verfahrens liegt in der Tatsache, daß keine weiteren, kontaminierenden hochmolekularen Proteine vorliegen. Durch einen einfachen größenausschlußchromatographischen Reinigungsschritt können sodann die gelösten, niedermolekularen kontaminierenden Proteine von dem 168 kD-Protein getrennt werden.

Der bevorzugte Puffer I enthält neben CHAPS in einer Konzentration von etwa 0,1-10% (w/v) weiterhin 100-600 mM NaCl, einen Komplexbildner, ein Antireduktans und einen Proteaseinhibitor in einem neutralen Phosphatpuffer, bevorzugt jedoch CHAPS in einer Konzentration von etwa 1% (w/v), 400 mM NaCl, 1 mM EDTA, 5 mM 2-Mercaptoethanol, 0,6 mM PMSF und 50 mM Natriumphosphatpuffer, pH 6,75. Diese Pufferkomponenten, die an sich üblicherweise verwendet werden, unterstützen in den genannten Mengen die Lösungswirkung des CHAPS.

Der bevorzugt verwendete Puffer II enthält neben dem nonionischen Detergenz Octylglucosid in einer Konzentration von etwa 0,1-10% (w/v), 100-600 mM NaCl, einen Komplexbildner, ein Antireduktans und einen Proteaseinhibitor in einem neutralen Phosphatpuffer, wobei Octylglucosid bevorzugt in einer Konzentration von etwa 2% (w/v) enthalten ist, sowie 400 mM NaCl, 1 mM EDTA, 5 mM 2-Mercaptoethanol, 0,6 mM PMSF und 50 mM Natriumphosphatpuffer, pH 6.75. Auch für das nonionische Detergenz Octylglucosid ist im vorliegenden Verfahren dieses Puffergemisch gut geeignet.

Um die Lösungswirkung der ersten Behandlungsschritts der ganzen Zellen von M.pneumoniae zu

3

unterstützen, können die mit CHAPS vorbehandelten Zellen mit Ultraschall behandelt werden, bevorzugt während einer Dauer von 1,5 min bei 0°C. Die Ultraschallbehandlung fördert die Auflösung der Zellwandbestandteile, wodurch zellwandgebundene Proteine verstärkt freigesetzt werden können.

Um das nach der Behandlung mit CHAPS sowie durch Ultraschall entstandene Homogenat in Fraktionen zu trennen, von denen eine das gewünschte 168 kD-Protein enthält, wird das Homogenat bevorzugt etwa 40 min bei 25 000 x g und 4°C zentrifugiert. In dem Pellet ist das gewünschte 168 kD-Protein enthalten. Das Pellet wird sodann gewaschen und in den obenbeschriebenen Puffer II aufgenommen. Hieran kann nochmal eine kurze Ultraschallbehandlung anschließen.

Das in dem Puffer II aufgenommene Pellet wird bei Raumtemperatur 5 min geschüttelt und daran anschließend nocheinmal zentrifugiert.

Der auf diese Weise erfolgten Extraktion des 168 kD-Proteins mit Octylglucosid, das im Puffer II enthalten ist, kann ein etwa 5 minütiges Schütteln bei Raumtemperatur der erhaltenen Suspension folgen, woran sich wiederum eine 40-minütige Zentrifugation bei 25 000 x g anschließt. Der erhaltene, opake Überstand enthält das 168 kD-Protein und kann für die anschließende Größenausschlußchromatographie gefiltert werden, beispielsweise durch eine 0,45 μm Membran.

Die Größenausschlußchromatographie kann bevorzugt mittels einer Superose 6 prep Grad-Säule erfolgen. Das 168 kD-Protein kann aus dieser Säule sodann mit einem SDS-Natriumphosphat-Puffer bei einem pH-Wert von etwa 6,75 in hoher Reinheit, großer Menge und nativem Zustand eluiert werden.

Die Erfindung wird in folgenden anhand der Figuren und Beispiele detailliert erläutert.

Es zeigen:

Fig. 1

Ein Serva-Blau-markiertes Polypeptidmuster, das nach SDS-PAGE Trennung der Proteine von M.pneumoniae erhalten wurde, wobei die Solubilisierung mit verschiedenen Detergenzien erfolgte.

Je 200 μg Zellprotein wurden 1,5 Minuten lang in Anwesenheit des jeweiligen Detergenz mit Ultraschall behandelt und anschließend weitere 5 Minuten bei Raumtemperatur inkubiert. Die Suspensionen werden bei 100 000 x g zentrifugiert und die erhaltenen Überstände werden für die SDS-PAGE präpariert. Es zeigen die Säulen

      a) 2% SDS
      b) 1% Triton X-100, 0,2% DOC, 0,1% SDS (TDSET)
      c) 1% CHAPS
      d) 1% Octylglucosid

Fig. 2

Das Solubilisieren des 168 kD-Proteins mit verschiedenen Konzentrationen des Octylglucosids.

M.pneumoniae-Zellen werden mit CHAPS-Puffer behandelt, zentrifugiert, die gewaschenen Pellets (je 100 μg Protein) werden in Puffer suspendiert, der verschiedene Konzentrationen des Octylglucosids enthält. Nach dem Zentrifugieren wird der Überstand für die SDS-PAGE präpariert und das Gel (8,25% T) geladen. Die fixierten und Serva-Blau markierten Gele werden bei 575 nm densitomentriert und die Bereiche der Peaks, die zu den markierten Banden des 168 kD-Proteins korrespondieren, werden gegen die Konzentration des Detergenz aufgetragen.

Fig. 3

Größenausschlußchromatographie des Octylglucosid-Solubilisats des 168 kD-Proteins von M.pneumoniae

1 ml Octylglucosid-Solubilisat des 168 kD-Proteins wird auf eine Säule (1,6 x 50 cm), die Superose 6 prep Grad enthält, aufgetragen. 1,2 ml Fraktionen werden mit 0,1% SDS in 100 mM Natriumphosphat mit pH 6,75 in einer Rate von 0,2 ml/min. eluiert.

Fig. 4

4

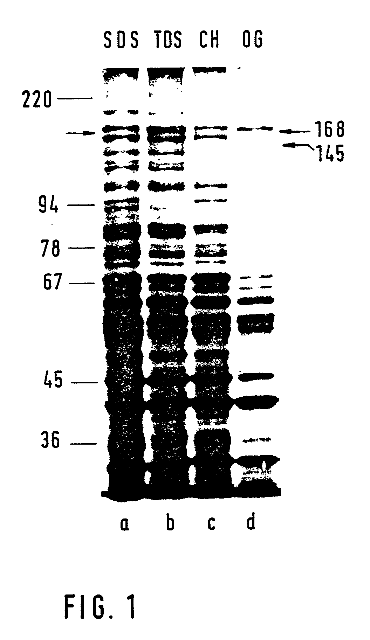

Polypeptidmuster nach SDS-PAGE und Serva-Blau-Markierung der Fraktionen, die durch Größenausschlußchromatographie des rohen 168 kD-Proteins erhalten wird. Die experimentellen Bedingungen sind dieselben, wie sie in Fig. 1 beschrieben sind. Es zeigen:

| Reihe 2: | gesamtes Zellprotein (200 µg) |
| Reihe 15: | 168 kD-Protein |
| Reihen 3-14: | verschiedene Fraktionen der Größenausschlußchromatographie. |

Die in der gesamten Beschreibung verwendeten Abkürzungen haben die folgende Bedeutung:

| SDS | Natriumdodecylsulfat |
| PAGE | Polyacrylamidgelelectrophorese |
| EDTA | Ethylendiamintetraacetat |
| DOC | Deoxycholat |
| HLB | Hydrophile-Lipophile-Balance |
| TDSET | Triton-DOC-SDS-EDTA-Tris-Puffer |
| CHAPS | 3-(3-Cholamidopropyl)dimethylammonio-3-Propansulfonat |
| PMSF | Phenylmethansulfonylfluorid |

## Beispiel 1

### Kultur von Mycoplasma pneumoniae

Ein Stamm FH von M.pneumoniae wird bei 37° in Roux-Flaschen in Hayflicksmedium (Hayflick L, Tex Rep Biol Med, 23; Suppl., S. 285-303, Cell cultures and mycoplasmas (1965)) gezüchtet. Die Zellen werden nach 48 Stunden geerntet und bei -70° aufbewahrt.

## Beispiel 2

### Isolieren des 168 kD-Proteins

Zellen von M.pneumoniae, hergestellt, wie im Beispiel 1 beschrieben, werden in 5 ml eines Puffers I (1% CHAPS, 400 mM NaCl, 1 mM EDTA, 1 mM 2-Mercaptoethanol, 0,6 mM PMSF, 50 mM Natriumphosphatpuffer mit einem pH-Wert von 6,75) suspendiert und für eine Dauer von 1,5 min bei 0°C ultrabeschallt. Das Homogenat wird 40 min lang bei 25 000 x g bei 4°C zentrifugiert.

Alle weiteren Verfahrensschritte werden bei Raumtemperatur duchgeführt.

Das gewaschene Pellet wird in 1 ml des Puffers II (2% Octylglucosid, 400 mM NaCl, 1 mM EDTA, 5 mM 2-Mercaptoethanol, 0,6 mM PMSF, 50 mM Natriumphosphatpuffer mit einem pH-Wert von 6,75) durch kurze Ultrabeschallung resuspendiert. Die erhaltene Suspension wird 5 min lang bei Zimmertemperatur geschüttelt und anschließend 40 min lang bei 25 000 x g zentrifugiert. Der opake Überstand enthält das rohe 168 kD-Protein. Der Überstand wird für den darauffolgenden Chromatographieschritt durch Filtrieren durch eine 0,45 µm Membran (Spartan, Schleicher und Schüll, Kassel/BRD) präpariert.

## Beispiel 3

### Größenausschlußchromatographie

Das Octylglucosidsolubilisat des rohen 168 kD-Proteins wird auf eine Säule (1,6 x 50 cm), enthaltend Superose 6 prep Grad (Deutsche Pharmacia AG GmbH, Freiburg/BRD) aufgetragen. Das Eluieren wird mit einer Durchflußrate von 0,2 ml/min. mit einem Elutionspuffer (0,1% SDS, 100 mM Natriumphosphatpuffer mit einem pH-Wert von 6,75) durchgeführt und es werden Fraktionen von 1,2 ml gesammelt.

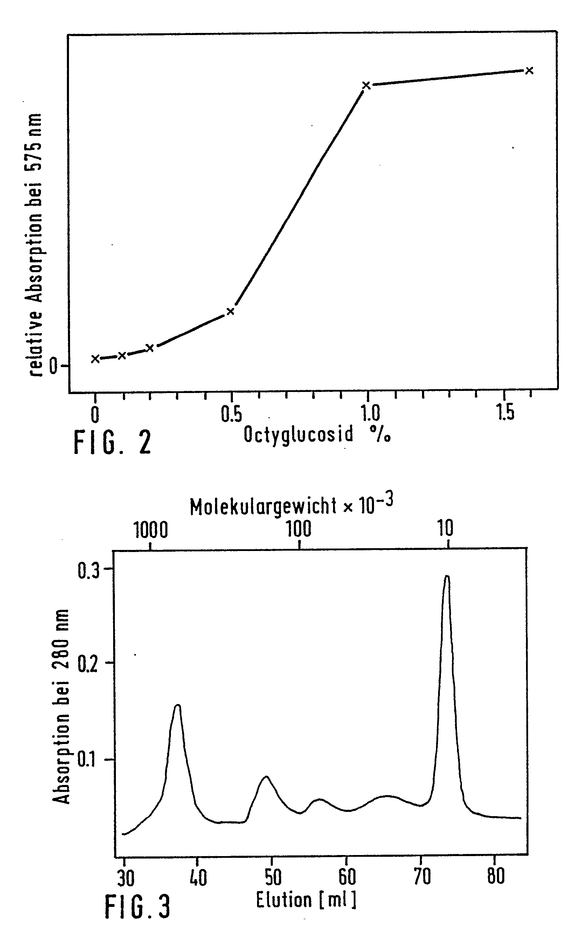

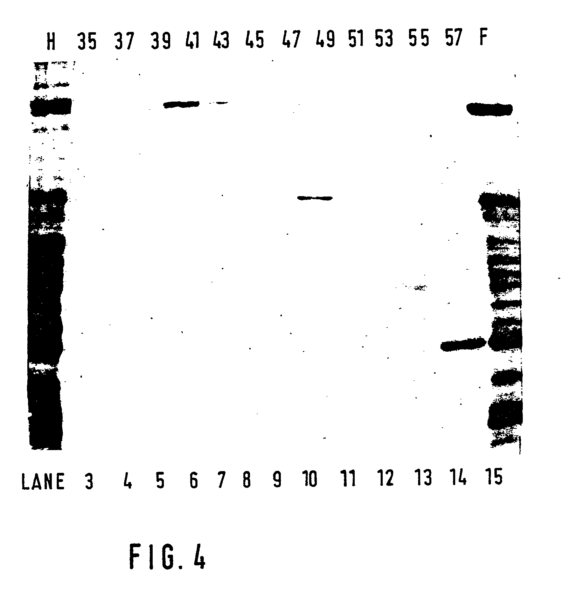

Fig. 3 zeigt ein Elutionsdiagramm, das zu Polypeptiden mit Molekulargewichten im Bereich von 200 000 bis 10 000 korrespondiert. Aliquots der Fraktionen werden durch SDS- PAGE (Fig. 4) analysiert. Obwohl in einigen Präparationen der erste Peak, der zum Ausschlußvolumen korrespondiert, etwa bis zu 10 fach höher war als in dem hier gezeigten Chromatogramm, enthielten die korrespondierenden Fraktionen kein Protein (Reihe 3, Fig. 4). Diese Absorption ist vermutlich bedingt durch die Anwesenheit von gemischten Mizellen in dem opaken Überstand, der auf die Säule gegeben wird. Der zweite Peak enthält das gut gelöste 168 kD-Protein, wie es aus den Reihen 7 bis 9 in Fig. 4 ersichtlich ist. Die Menge an 168 kD-Protein, die durch das Verfahren erhalten werden kann, liegt bei 40% oder 300 μg 168 kD-Protein aus 1 g Naßzellgewicht des Pellets.

Beispiel 4

SDS-PAGE

Die gesammelten Proteinfraktionen werden durch SDS-PAGE in 1,5 mm dicken Slabgelen (18 x 14 cm) mit einer Gel-konzentration von 8,75 % T in einem diskontinuierlichen Puffersystem (Laemmli U.K., Nature 227: S. 680-685, Cleavage of structural proteins during the assembly of the head of bacteriophage T4 (1970)) analysiert.

Beispiel 5

Proteinassay in verdünnter Detergenzienlösung

Das Protein wird durch simultane Extraktion von Detergenzien präzipitiert (Wessel D, Flügge UI; Anal. Biochem. 138; S.141-143, A method for the quantitative recovery of protein in dilute solution in the presence of detergents and lipids (1984)) und das gesammelte Protein wird in einem modifizierten Lowry-Verfahren bestimmt (Peterson GL, Anal.Biochem. 83; S.346-356, A simplification of the protein assay method of Lowry et al which is more generally applicable (1977)).

Vergleichsversuch 1

Die M.pneumoniae-Zellen, hergestellt, wie im Beispiel 1 beschrieben, werden in Aliquots mit Puffern behandelt, die je 1% CHAPS oder 1% Octylglucosid oder die Detergenzienmischung TDSET (1% v/v) Triton X 100, 0,2% (w/v) Deoxycholat, 0,1% (w/v) SDS, 10 mM EDTA, 10 mM Tris-HCl mit einem pH-Wert von 7,8), wie bei Leith (1984), a.a.O.) beschrieben, behandelt. Die erhaltenen Suspensionen werden zentrifugiert und die so erhaltenen Über stände in SDS-PAGE analysiert, wie in Fig. 1 beschrieben.

Fig. 1 zeigt in Reihe a das Polypeptidmuster, das nach SDS-PAGE und Serva-Blau-Markierung erhalten wird; es zeigt praktisch das gesamte Zellprotein. Die Molekulargewichte sind am linken Rand der Fig. 1 angezeigt und die Position des 168 kD-Proteins durch einen Pfeil bezeichnet. Die Reihen b bis d zeigen die Polypeptidmuster der Proteinfraktionen, die nach Solubilisieren in Anwesenheit von TDSET, bzw. 1% CHAPS, bzw. 1% Octylglucosid, erhalten werden.

Es zeigt sich, daß TDSET gute Lösungseigenschaften sowohl für Proteine geringen Molekulargewichts als auch für das 168 kD-Protein als auch für ein weiteres wesentliches Protein von M.pneumoniae mit einem Molekulargewicht von 145 kD hat. Die beiden Proteine mit den Molekulargewichten 145 kD und 168 kD konnten, wie eine spätere Größenausschlußchromatographie zeigte, nicht voneinander getrennt werden.

Das Detergenz Octylglucosid zeigt gute Lösungseigenschaften für das 168 kD-Protein, schlechtere für das 145 kD-Protein. Im Falle des Detergenz CHAPS zeigt sich, daß es ein sehr effizientes Detergenz für die Solubilisierung des 145 kD-Proteins ist, jedoch eine geringere Lösungskraft für das 168 kD-Protein hat.

Vergleichsversuch 2

Bestimmen der wirksamen Konzentration des Octylglucosids.

Fig. 2 zeigt die Ergebnisse, die durch Behandlung der mit CHAPS vorbehandelten M.pneumoniae-Zellen mit einem Puffer II, wie er in Beispiel 2 beschrieben wurde, wobei Octylglucosid in verschiedenen Konzentrationen zwischen 0 und 2% enthalten ist. Fig. 2 zeigt, daß Konzentrationen des Octylglucosids über 1% ausreichen für die Solubilisierung des 168 kD-Proteins. Um Toleranzbereiche für verschiedene Proteinkonzentrationen sicherzustellen und ein Verhältnis des Detergenz/Protein von mindestens 5 zu erhalten, ist eine Konzentration von 2% des Octylglucosids besonders geeignet, da dann Proteinkonzentrationen bis etwa 4 mg/ml erlaubt sind.

Das SDS-PAGE-Polypeptidmuster des 2%igen Octylglucosid-Solubilisats, das aus Pellets erhalten wird, die mit CHAPS vorbehandelt werden, zeigt Reihe 15 in Fig. 4.

Durch die erfindungsgemäß durchgeführte fraktionierte Solubilisierung des 168 kD-Proteins wird eine Anreicherung um das 20-fache, gemessen an etwa 1,5% des gesamten Zellproteins von M.pneumoniae erreicht. Diese Werte können über Densitometrie erhalten werden. Das mit dem erfindungsgemäßen Verfahren hergestellte 168 kD-Protein enthält keine kontaminierenden hochmolekularen Proteine.

Durch das fraktionierte Lösen des gewünschten 168 kD-Proteins mit den erfindungsgemäßen Verfahrensschritten unter Verwendung des zwitterionischen Detergenz CHAPS sowie des nonionischen Detergenz Octylglucosid ist eine bis zu 20-fache Anreicherung des gewünschten Proteins möglich, wobei verbleibende Verunreinigungen ein weitaus geringeres Molekulargewicht als das gewünschte Protein aufweisen und somit durch einem einfachen Gelfiltrationsschritt getrennt werden können. Mit dem erfindungsgemäßen Verfahren ist es somit nicht nötig, das 168 kD-Protein denaturierenden Agenzien auszusetzen, so daß das Protein in seiner nativen Form erhalten wird.

## Ansprüche

1. Verfahren zum Reinigen eines 168 kD-Proteins von Mycoplasma pneumoniae, **dadurch gekennzeichnet,** daß ganze M.pneumoniae-Zellen mit einem zwitterionsichen Detergenz, bevorzugt 3-(3-Cholamidopropyl) dimethylammonio-3-Propansulfonat (CHAPS) behandelt werden, sodann aus dem erhaltenen Pellet das 168 kD-Protein mit einem nonionischen Detergenz, bevorzugt Octyl-ß-D-glucopyranosid (Octylglucosid) extrahiert und schließlich weitere Verunreinigungen durch Größenausschlußchromatographie entfernt werden.

2. Verfahren nach Anspruch 1, **dadurch gekennzeichnet,** daß CHAPS in einem Puffergemisch (Puffergemisch I) in einer Konzentration von etwa 0,1-10% (w/v), bevorzugt 1% (w/v), enthalten ist.

3. Verfahren nach Anspruch 1, **dadurch gekennzeichnet,** daß das Octylglucosid in einem Puffergemisch (Puffergemisch II) in einer Konzentration von etwa 0,1-10% (w/v), bevorzugt 1-2% (w/v), enthalten ist.

4. Verfahren nach Anspruch 2, **dadurch gekennzeichnet,** daß der Puffer I 0,1-10% (w/v) CHAPS, 100-600 mM NaCl, einen Komplexbildner, ein Antireduktans und einen Proteaseinhibitor in einem neutralen Phosphatpuffer enthält, bevorzugt 1% (w/v) CHAPS, 400 mM NaCl, 1 mM EDTA, 5 mM 2-Mercaptoethanol, 0,6 mM PMSF und 50 mM Natriumphosphatpuffer pH 6,75 enthält.

5. Verfahren nach Anspruch 3, **dadurch gekennzeichnet,** daß der Puffer II 0,1-10% (w/v) Octylglucosid, 100-600 mM NaCl, einen Komplexbildner, ein Antireduktans und einen Proteaseinhibitor in einem neutralen Phosphatpuffer enthält, bevorzugt 2% (w/v) Octylglucosid, 400 mM NaCl, 1mM EDTA, 5 mM 2-Mercaptoethanol, 0,6 mM PMSF und 50 mM Natriumphosphatpuffer pH 6,75 enthält.

6. Verfahren nach mindestens einem der vorhergehenden Ansprüche, **dadurch gekennzeichnet,** daß nach der Behandlung mit CHAPS die Zellen mit Ultraschall, bevorzugt etwa 1,5 Min bei 0°C behandelt werden.

7. Verfahren nach Anspruch 6, **dadurch gekennzeichnet,** daß das nach Ultraschallbehandlung erhaltene Homogenat zentrifugiert wird, bevorzugt etwa 40 min. bei 25000 x g und 4°C.

8. Verfahren nach mindestens einem der vorhergehenden Ansprüche, **dadurch gekennzeichnet,** daß nach der Extraktion mit Octylglucosid die erhaltene Suspension etwa 5 min. bei Raumtemperatur geschüttelt und anschließend 40 min. bei 25 000 x g zentrifugiert und der erhaltene Überstand, der das 168 kD-Protein enthält, filtriert wird.

9. Verfahren nach mindestens einem der vorhergehenden Ansprüche, **dadurch gekennzeichnet,** daß die Größenausschlußchromatographie mittels einer Superose 6 prep Grad Säule erfolgt und das 168 kD-Protein mit einem SDS-Natriumphosphatpuffer mit einem neutralen pH-Wert , bevorzugt 6,75, eluiert wird.

FIG. 1

FIG. 2

relative Absorption bei 575 nm

Octyglucosid %

FIG.3

Molekulargewicht × 10⁻³

Absorption bei 280 nm

Elution [ml]

FIG. 4